## Europäisches Patentamt
## European Patent Office
## Office européen des brevets

(19)

(11) Publication number: **0 002 749**
**B2**

(12) # NEW EUROPEAN PATENT SPECIFICATION

(45) Date of publication of the new patent specification: **12.08.87**

(21) Application number: **78101715.7**

(22) Date of filing: **15.12.78**

(51) Int. Cl.⁴: **C 07 C 51/255**

(54) Process for producing aromatic monocarboxylic acid.

(30) Priority: 26.12.77 JP 157071/77
26.12.77 JP 157072/77

(43) Date of publication of application:
**11.07.79 Bulletin 79/14**

(45) Publication of the grant of the patent:
**19.10.83 Bulletin 83/42**

(45) Mention of the opposition decision:
**12.08.87 Bulletin 87/33**

(84) Designated Contracting States:
**DE FR GB SE**

(56) References cited:
DE-A-2 033 406
DE-A-2 258 503
DE-A-2 703 161
DE-A-2 703 161
DE-B-1 220 408
DE-B-1 235 887
DE-B-1 418 852
DE-C- 767 366
US-A-2 245 528
US-A-2 276 774
US-A-3 030 414
US-A-3 649 681

(73) Proprietor: IHARA CHEMICAL INDUSTRY CO., LTD.
4-26, Ikenohata 1-chome
Taitoh-ku, Tokyo (JP)

(72) Inventor: Shigeyasu, Motoo
1135-23, Shinhamacho-Koh
Matsuyama-shi Ehime-ken (JP)
Inventor: Yamazaki, Hatsutaro
4200-7, Takayamacho
Matsuyama-shi Ehime-ken (JP)
Inventor: Kitamura, Takehiko
49-53, Shinhamacho-Otsu
Matsuyama-shi Ehime-ken (JP)
Inventor: Doi, Norimasa
552-1, Kitasayacho
Matsuyama-shi Ehime-ken (JP)
Inventor: Fukudome, Toshiyuki
552-1, Kitasayacho
Matsuyama-shi Ehime-ken (JP)

(74) Representative: Wächtershäuser, Günter, Dr.
Tal 29
D-8000 München 2 (DE)

(56) References cited:
US-A-3 970 696
US-A-4 007 223

EP 0 002 749 B2

Courier Press, Leamington Spa, England.

## Description

The present invention is concerned with a process for producing an aromatic monocarboxylic acid by oxidizing a benzene derivative having one alkyl group in the liquid phase with molecular oxygen or a molecular oxygen-containing gas in the presence of a catalyst at an increased temperature.

Description of the Prior Arts

Heretofore, it has been known that aromatic monocarboxylic acids having one carboxylic acid group can be produced by oxidizing a benzene derivative having one alkyl group with molecular oxygen or a molecular oxygen-containing gas in the presence of an oil soluble metal salt catalyst without any solvent.

However, in the conventional reaction in the absence of a solvent, it is necessary to accept a low conversion of the benzene derivative in order to increase the selectivity to the object aromatic monocarboxylic acid.

When the conversion of the benzene derivative is increased, the selectivity to the object aromatic monocarboxylic acid is remarkably decreased and large amounts of by-products such as colored materials are formed.

When an oxidation in liquid phase is carried out in the absence of a solvent, the reaction involves a free-radical chain reaction whereby an absorption of oxygen is not found at the initial stage, and a severe reaction occurs suddenly at the initiation of the absorption of oxygen. It has also been known that an aromatic monocarboxylic acid is produced by oxidizing a benzene derivative in liquid phase with molecular oxygen or molecular oxygen-containing gas in the presence of a metal catalyst in a large amount of a solvent such as two or more times by weight of a solvent. In this process the conversion of the benzene derivative and the selectivity to the aromatic monocarboxylic acid are increased. However, the solubility of the aromatic monocarboxylic acid in the solvent is relatively high whereby it is difficult to recover the object aromatic monocarboxylic acid in high yield from the solvent. Therefore the yield of the object aromatic monocarboxylic acid is disadvantageously low. Further the costs for recovering the solvent and the loss of the solvent are high.

A process for oxidizing o-bromo or o-chlorotoluene in liquid phase with oxygen in acetic acid as solvent in the presence of a catalyst of cobalt acetate and hydrogen bromide at 90°C for 2 hours has been disclosed in Canadian Journal of Chemistry 43, 1306 to 1317 (1965). In this process, relatively large amounts of the solvent and the catalyst are used and the selectivity to the object aromatic monocarboxylic acid is low.

Summary of the Invention

It is an object of the present invention to provide a process for producing an aromatic monocarboxylic acid having high quality in high yield in a smooth reaction with a high conversion of the benzene derivative having an alkyl group and a high selectivity to the object aromatic monocarboxylic acid.

The foregoing object is attained by a process for producing an aromatic monocarboxylic acid by oxidizing a benzene derivative having one alkyl group in the liquid phase with molecular oxygen or a molecular oxygen-containing gas in the presence of a catalyst at an increased temperature, which is characterized in that the reaction is conducted in the presence of a lower aliphatic monocarboxylic acid with two to four carbon atoms in an amount of from 0.15 to 0.5 times by weight of the amount of said benzene derivative present in the reaction system at a temperature ranging from a temperature adequate to maintain the resulting aromatic monocarboxylic acid in liquid form to 180°C, said catalyst comprising a cobalt and manganese compound.

Detailed Description of the Preferred Embodiments

In spite of the use of a reaction system containing substantially no solvent, the reaction can be smoothly performed with high conversion, high selectivity, high product quality and high yield.

The lower aliphatic monocarboxylic acids incorporated in the reaction system of the process of the present invention include acetic acid, propionic acid and butyric acid and acetic acid is preferably used. The amount of the lower aliphatic monocarboxylic acid is 0.15 to 0.5 times by weight based on the benzene derivative.

When the amount of the lower aliphatic monocarboxylic acid is less than 0.05 times by weight based on the benzene derivative, the oxidation is not easily initiated and a severe oxidation sets in suddenly after the initiation and various by-products such as colored materials are produced and the selectivity is too low.

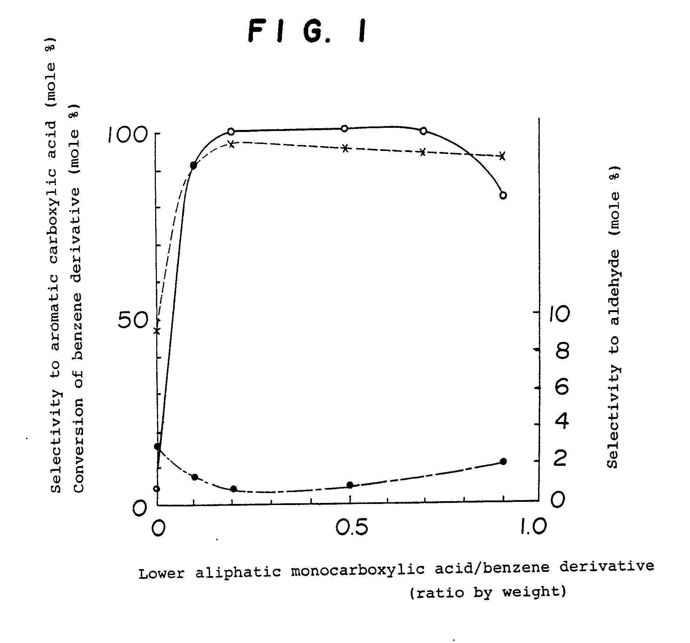

Effects of the amount of the lower aliphatic monocarboxylic acid to the conversion of the benzene derivative, the selectivity to the object aromatic monocarboxylic acid and the selectivity to the aldehyde formed as by-product are shown in a graph of Figure 1.

When the amount of the lower aliphatic monocarboxylic acid is higher or lower than the above-defined range, the conversion of the benzene derivative and the selectivity to the object aromatic monocarboxylic acid are low whereas the selectivity to the aldehyde as the by-product is high.

It has been obtained under the conditions of the following Example 1, except varying the amount of the lower aliphatic monocarboxylic acid. In Figure 1, the full line shows the conversion of the benzene

2

derivative, and dotted line shows the selectivity to the object aromatic monocarboxylic acid and the dotted chain line shows the selectivity to the aldehyde.

Suitable benzene derivatives having one alkyl group include alkylbenzenes such as toluene, ethylbenzene, n-propylbenzene and i-propylbenzene; substituted alkylbenzenes having one or more inert substituent such as halogen atoms, nitro group, carboxymethyl group and methoxy group on the benzene ring such as alkyl halobenzenes e.g. o-, m- or p-chlorotoluene and o-, m- or p-bromotoluene; alkyl nitrobenzenes e.g. o-, m- or p-nitrotoluene; alkyl carboxymethylbenzenes such as o-, m- or p-carboxymethyltoluene; and alkyl methoxybenzenes e.g. o-, m- or p-methoxytoluene.

The heavy metal compound catalysts used in the present invention are catalysts having heavy metal components of cobalt and manganese as main components or catalysts having heavy metal components of cobalt and manganese main components with a second or third additional component of nickel or cerium.

The heavy metal compound is preferably in the form of an aliphatic carboxylic acid salt or a halide which is soluble in the lower aliphatic monocarboxylic acid.

It is possible to incorporate a reaction accelerator such as halogen, halogen compounds, aldehydes and ketones. When bromine is incorporated as the reaction accelerator, superior results may be attained. The incorporation of bromine is preferable.

The amount of the catalyst is usually more than 0.005 weight % preferably 0.03 to 2 weight % especially 0.05 to 0.5 weight % as the metal component based on the benzene derivative.

It is especially preferable to use a ratio of Mn/Co of 0.5 to 500 weight %. The total amount of the reaction accelerator such as halogens, halogen compounds, aldehydes and ketones is preferably in the range of 0.05 to 30 weight % based on the benzene derivative. The amount of the halogen compound is preferably in the range of 0.05 to 5.0 especially 0.1 to 1.0 weight % as halogen based on the benzene derivative.

The effect of the present invention can be further improved by selecting the kinds and the amounts of the catalyst component and reaction accelerator.

In the process of the present invention, the reaction is preformed by incorporating the benzene derivative, the heavy metal compound catalyst, if necessary, with the reaction accelerator, the lower aliphatic monocarboxylic acid and molecular oxygen or molecular oxygen-containing gas in the reaction system.

It is necessary to perform the reaction at a temperature higher than the temperature for maintaining the resulting aromatic monocarboxylic acid in liquid form in the reaction system, preferably higher than the melting point of the resulting aromatic monocarboxylic acid.

When the reaction temperature is lower than the temperature for maintaining the resulting aromatic monocarboxylic acid in liquid form, a part of the resulting aromatic monocarboxylic acid solidifies and homogeneous reaction can not be performed. Further side-reactions increase and the yield of the object aromatic monocarboxylic acid is reduced.

In general, the minimum temperature for maintaining the aromatic monocarboxylic acid in liquid form is slightly lower than the melting point of the resulting aromatic monocarboxylic acid when the amount of the lower aliphatic monocarboxylic acid is near the lower limit of the defined range whereas it is about 40°C lower than the melting point of the resulting aromatic monocarboxylic acid when the amount of the lower aliphatic monocarboxylic acid is near upper limit of the defined range.

In the process of the present invention, the reaction pressure can be the sum of the pressure for maintaining the reaction system in liquid form and the partial pressure of the molecular oxygen or molecular oxygen-containing gas. The partial pressure of the molecular oxygen or molecular oxygen-containing gas is preferably in the range of 1 to 30 atm. especially 5 to 20 atm. When the partial pressure of molecular oxygen or molecular oxygen-containing gas is too low, the reaction is too slow and side-reactions occur. On the other hand, there is no advantageous effect for the reaction velocity and the quality of the resulting aromatic monocarboxylic acid if the partial pressure is increased over 30 atm.

The molecular oxygen-containing gas is preferably air as it is economical.

In the feeding of the molecular oxygen or molecular oxygen-containing gas into the reaction system, it is preferable to control the concentration of oxygen in the waste gas discharged from the reaction system in a non-explosive range.

When chlorotoluene is oxidized to obtain chlorobenzoic acid, the catalyst comprising 0.025 to 0.5 wt.% preferably 0.04 to 0.3 wt.% as Co of cobalt component based on the lower aliphatic monocarboxylic acid, 0.5 to 250 wt.% preferably 1 to 150 wt.% as Mn of manganese component based on cobalt compound and 0.07 to 3 wt.% preferably 0.10 to 1.0 wt.% as Br of bromine compound based on the lower aliphatic monocarboxylic acid is preferably used to prevent a removal of the chlorine atom. The bromine component can also be inorganic or organic bromide compounds such as sodium bromide, ammonium bromide, methyl bromide and bromoform which is soluble in the lower aliphatic monocarboxylic acid.

When chlorotoluene is oxidized to obtain chlorobenzoic acid, the reaction is preferably performed at 120 to 180°C under a pressure of 3 to 30 atm.

The bromine compound is preferably hydrogen bromide, cobalt bromide or manganese bromide which is the catalyst itself or a combination.

The reaction time is preferably 2 to 8 hours to obtain the object aromatic monocarboxylic acid having high quality in high yield.

The reaction can be performed in a batch system, a semi-continuous system or a continuous system.

The lower aliphatic monocarboxylic acid and the catalyst components are separated from the reaction mixture by conventional or other suitable methods to obtain the object aromatic monocarboxylic acid.

For example, the reaction mixture is cooled and the solid component is separated, washed and dried. The reaction mixture is fed to a distillator and light components such as the lower aliphatic monocarboxylic acid are distilled off as an initial fraction and the solid components such as the catalyst and high boiling components are discharged from the bottom of the reactor as the bottom residue and the object aromatic monocarboxylic acid is distilled as the middle fraction.

In accordance with the process of the present invention, the object aromatic monocarboxylic acid is obtained with high conversion and high selectivity and the amount of the by-products contained in the reaction mixture after the reaction is small whereby the object aromatic monocarboxylic acid having high quality can be easily obtained from the reaction mixture by a simple operation such as distillation. The operation for obtaining the object aromatic monocarboxylic acid from the. reaction mixture can be simplified in comparison with the conventional process.

In accordance with the process of the present invention, the object aromatic monocarboxylic acid can be produced in high conversion and high selectivity even though the reaction condition is relatively mild. Accordingly, the process can be advantageously applied to the oxidation of an alkylbenzene having a substituent which is easily removed under severe reaction conditions.

The present invention will be further illustrated by examples and references which are provided for the purpose of illustration only.

In the examples and references, the benzene derivatives had a purity of greater than 99% and the contents of components in the reaction mixture were determined by a gas chromatography.

From the contents of the components in the reaction mixture, conversions of the benzene derivative, selectivities to the object aromatic monocarboxylic acid and yields thereof and selectivities to the aldehyde are calculated.

The reaction products beside the object aromatic monocarboxylic acid and the aldehyde are considered as the other intermediates and by-products.

(1) Conversion of benzene derivative as the starting material (mole %)

$$= \left(1 - \frac{\text{unreacted benzene derivative}}{\text{charged benzene derivative}}\right) \times 100$$

(2) Selectivity to object aromatic monocarboxylic acid (mole %)

$$= \left[\frac{\text{moles of aromatic monocarboxylic acid}}{(\text{moles of charged benzene derivative}) - (\text{moles of unreacted benzene derivative})}\right] \times 100$$

(3) Selectivity to aldehyde (mole %)

$$= \left[\frac{\text{moles of aldehyde}}{(\text{mole of charged benzene derivative}) - (\text{moles of unreacted benzene derivative})}\right] \times 100$$

(4) Yield of aromatic monocarboxylic acid (mole %)

$$= \frac{(\text{conversion of benzene derivative}) \times (\text{selectivity to aromatic monocarboxylic acid})}{100}$$

Example 1

In a reactor made of titanium equipped with a reflux condenser, a stirrer, a gas inlet and a gas outlet, 700 g of o-chlorotoluene. 140 g of acetic acid, 3.9 g of cobalt bromide (0.1 wt.% as Co to o-chlorotoluene), 0.06 g of manganese acetate (2 wt.% as Mn to cobalt component) were charged and thoroughly stirred. Then, nitrogen gas was charged from the gas inlet to give the pressure of 10 Kg/cm²G and the reactor was heated to 145°C and then, air was fed at a flow rate of 6.5 N liter/min., and the reaction was performed under

4

the pressure of 10 Kg/cm$^2$ (Gauge) at 145°C until an absorption of oxygen was finished. It took about 4.5 hours for the reaction. After the reaction, the reaction mixture was cooled and taken out. The total weight of the reaction mixture was 1052 g.

Contents of o-chlorotoluene, o-chlorobenzoic acid and o-chlorobenzaldehyde were measured to give the following values.

Conversion of o-chlorotoluene, selectivity to o-chlorobenzoic acid, selectivity to o-chlorobenzaldehyde and yield of o-chlorobenzoic acid were determined. The results are shown in Table together with the reaction conditions.

Contents of components in reaction mixture

| | |
|---|---|
| o-chlorotoluene | 0.07 wt.% |
| o-chlorobenzoic acid | 79.5 wt.% |
| o-chlorobenzaldehyde | 0.43 wt.% |
| others (acetic acid, water, catalyst other by-product) | 20.0 wt.% |

Example 2, References 1 to 4

In accordance with the process of Example 1 except varying the amount of acetic acid as described below and varying the air flow rate as shown in Table 1, each reaction was performed for the reaction time as shown in Table 1.

In the References 1 and 2, the absorption of oxygen was not smooth after the initiation of the reaction. However, air was fed for 10 hours and the reaction was stopped.

Amount of acetic acid

| | |
|---|---|
| Example 2 | 350 g |
| Reference 1 | 490 g |
| Reference 2 | 70 g |
| Reference 3 | 0 g |
| Reference 4 | 630 g |

In these experiments, conversions of o-chlorotoluene, selectivities to o-chlorobenzaldehyde and yields of o-chlorobenzoic acid are shown in Table 1.

Example 3; References 5 to 7

In accordance with the process of Example 1 except varying the reaction temperature, the pressure and the air flow rate as shown in Table 1, each reaction was performed for the reaction time shown in Table 1.

In Reference 6, the absorption of oxygen was not smooth after the initiation of the reaction.

However, air was fed for 10 hours and the reaction was stopped.

In these experiments, conversions of o-chlorotoluene, selectivities to o-chlorobenzoic acid, selectivities to o-chlorobenzaldehyde and yields of o-chlorobenzoic acid are shown in Table 1.

Reference 8

In accordance with the process of Example 1, except using no manganese acetate, the reaction was performed for the reaction time of 6.2 hours.

In this experiment, conversion, selectivities and yield are shown in Table 1.

Example 4

In accordance with the process of Example 1 except using toluene as the starting material, and using 3.9 g of cobalt bromide (0.1 wt.% as Co to toluene) and 0.47 g of manganese acetate (15 wt.% as Mn to cobalt component) as the catalyst and feeding air at flow rate of 13.0 N liter/min. The reaction was performed for the reaction time of 3.0 hours.

In this experiment, conversion of toluene, selectivity to benzoic acid, selectivity to benzaldehyde and yield of benzoic acid were shown in Table 2.

Example 5

In accordance with the process of Example 1 except using m-nitrotoluene as the starting material and using 3.9 g of cobalt bromide (0.1 wt.% as Co to m-nitrotoluene) and 3.1 g of manganese acetate (100% as Mn to cobalt component) as the catalyst and feeding air at flow rate of 7.5 N liter/min., the reaction was performed for the reaction time of 3.5 hours.

In this experiment, conversion of m-nitrotoluene, selectivity to m-nitrobenzoic acid, selectivity to m-nitrobenzaldehyde and yield of m-nitrobenzoic acid are shown in Table 2.

Example 6

In accordance with the process of Example 1 except using toluene as the starting material and using 11.7 g of cobalt bromide (0.3 wt.% as Co to toluene) and 9.4 g of manganese acetate (100 wt.% as Mn to cobalt component) as the catalyst and reacting at 125°C under pressure of 7 Kg/cm$^2$ Gauge and feeding air at a flow rate of 7.5 N liter/min., the reaction was performed for the reaction time of 4.9 hours.

In this experiment, conversion of toluene, selectivity to benzoic acid, selectivity to benzaldehyde and yield of benzoic acid are shown in Table 2.

## Reference 9

In accordance with the process of Example 6, except reacting at 100°C under a pressure of 2.5 Kg/cm² Gauge for 10 hours and feeding air at a flow rate of 4.5 N liter/min., the reaction was performed.

In the process, the absorption of oxygen was not substantially found after the initiation of the reaction. However, air was fed for 10 hours and the reaction was stopped.

In this experiment, conversion, selectivities and yield are shown in Table 2.

## Example 7

In accordance with the process of Example 6 except using 0.19 g of manganese acetate (2 wt.% as Mn to cobalt component), the reaction was performed for the reaction time of 4.5 hours.

In this experiment, conversion, selectivities and yield are shown in Table 2.

## Example 8

In accordance with the process of Example 6 except using 3.9 g of cobalt bromide (0.1 wt.% as Co to toluene) and 9.4 g of manganese acetate (300 wt.% as Mn to cobalt component) as the catalyst and the reaction was performed for the reaction time of 4.0 hours.

In this experiment, conversion, selectivities and yield are shown in Table 2.

## Example 9

In accordance with the process of Example 1 except using cobalt acetate (0.1 wt.% as Co to o-chlorotoluene) and 0.06 g of manganese acetate (2 wt.% as Mn to cobalt component), and 6.1 g of hydrobromic acid (47 wt.% HBr aq. sol.) as the catalyst, the reaction was performed for the reaction time of 4.8 hours.

In the experiments, conversion, selectivities and yield are shown in Table 2.

## TABLE 1

|  | Exp. 1 | Exp. 2 | Ref. 1 | Ref. 2 | Ref. 3 |
|---|---|---|---|---|---|
| Starting material | CT | CT | CT | CT | CT |
| **Catalyst** | | | | | |
| Co %/starting material | 0.1 | 0.1 | 0.1 | 0.1 | 0.1 |
| Mn/Co (Wt. ratio) | 0.02 | 0.02 | 0.02 | 0.02 | 0.02 |
| Br%/starting material | 0.27 | 0.27 | 0.27 | 0.27 | 0.27 |
| **Amount of acetic acid** | | | | | |
| Acetic acid/starting material (Wt. ratio) | 0.2 | 0.5 | 0.7 | 0.1 | 0.0 |
| Reaction temperature (°C) | 145 | 145 | 145 | 145 | 145 |
| Reaction pressure (Kg/cm² (G)) | 10 | 10 | 10 | 10 | 10 |
| **Air flow rate** (N liter/min.) | 6.5 | 6.5 | 6.5 | 6.5 | 6.5 |
| Reaction hours (hrs.) | 4.5 | 4.4 | 5.2 | 7.2 | 10 |
| Conversion (mole %) | 99.9 | 99.9 | 97.8 | 90.5 | 4.4 |
| **Selectivities** | | | | | |
| to carboxylic acid (mole %) | 96.8 | 95.4 | 93.1 | 90.2 | 47.7 |
| to aldehyde (mole %) | 0.58 | 0.79 | 0.92 | 1.5 | 3.1 |
| Yield of object carboxylic acid (mole %) | 96.7 | 95.3 | 91.0 | 81.6 | 21 |

# 0 002 749

TABLE 1 (continued)

|  | Ref. 4 | Exp. 3 | Ref. 5 | Ref. 6 | Ref. 7 |
|---|---|---|---|---|---|
| Starting material | CT | CT | CT | CT | CT |
| Catalyst | | | | | |
| Co %/starting material | 0.1 | 0.1 | 0.1 | 0.1 | 0.1 |
| Mn/Co (Wt. ratio) | 0.02 | 0.02 | 0.02 | 0.02 | 0.02 |
| Br%/starting material | 0.27 | 0.27 | 0.27 | 0.27 | 0.27 |
| Amount of acetic acid | | | | | |
| Acetic acid/starting material (Wt. ratio) | 0.9 | 0.2 | 0.2 | 0.2 | 0.2 |
| Reaction temperature (°C) | 145 | 170 | 200 | 130 | 250 |
| Reaction pressure (Kg/cm$^2$ G) | 10 | 10 | 15 | 7 | 15 |
| Air flow rate (N liter/min.) | 4.5 | 10.0 | 13.0 | 4.5 | 13.0 |
| Reaction hours (hrs.) | 10 | 3.1 | 2.5 | 10 | 20 |
| Conversion (mole %) | 81.5 | 99.9 | 99.9 | 8.4 | 99.9 |
| Selectivities | | | | | |
| to carboxylic acid (mole %) | 91.5 | 95.2 | 88.1 | 83.5 | 72.1 |
| to aldehyde (mole %) | 20 | 0.43 | 0.35 | 6.4 | 0.35 |
| Yield of object carboxylic acid (mole %) | 74.6 | 95.1 | 88.0 | 7.0 | 72.0 |

CT=o-chlorotoluene

TABLE 2

|  | Ref. 8 | Exp. 4 | Exp. 5 | Exp. 6 |
|---|---|---|---|---|
| Starting material | CT | T | NT | T |
| Catalyst | | | | |
| Co %/starting material | 0.1 | 0.1 | 0.1 | 0.3 |
| Mn/Co (Wt. ratio) | 0 | 0.15 | 1.0 | 1.0 |
| Br%/starting material | 0.27 | 0.27 | 0.27 | 0.82 |
| Amount of acetic acid | | | | |
| Acetic acid/starting material (Wt. ratio) | 0.2 | 0.2 | 0.2 | 0.2 |
| Reaction temperature (°C) | 145 | 145 | 145 | 125 |
| Reaction pressure (Kg/cm$^2$ G) | 10 | 10 | 10 | 7 |
| Air flow rate (N liter/min.) | 6.5 | 13.0 | 7.5 | 7.5 |
| Reaction time (hrs.) | 6.2 | 3.0 | 3.5 | 4.9 |
| Conversion (mole %) | 91.5 | 99.9 | 99.1 | 99.0 |
| Selectivities | | | | |
| to carboxylic acid (mole %) | 92.1 | 95.8 | 94.8 | 96.5 |
| to aldehyde (mole %) | 2.7 | 0.48 | 0.66 | 0.44 |
| Yield of object carboxylic acid (mole %) | 84.2 | 95.7 | 94.0 | 95.5 |

7

TABLE 2 (continued)

| | Ref. 9 | Exp. 7 | Exp. 8 | Exp. 9 |
|---|---|---|---|---|
| Starting material | T | T | T | CT |
| Catalyst | | | | |
| Co %/starting material | 0.3 | 0.3 | 0.1 | 0.1 |
| Mn/Co (Wt. ratio) | 1.0 | 0.02 | 3.0 | 0.02 |
| Br%/starting material | 0.82 | 0.82 | 0.27 | 0.27 |
| Amount of acetic acid | | | | |
| Acetic acid/starting material (Wt. ratio) | 0.2 | 0.2 | 0.2 | 0.2 |
| Reaction temperature (°C) | 100 | 125 | 125 | 145 |
| Reaction pressure (Kg/cm$^2$ G) | 2.5 | 7 | 7 | 10 |
| Air flow rate (N liter/min.) | 4.5 | 7.5 | 7.5 | 6.5 |
| Reaction time (hrs.) | 10 | 4.5 | 4.0 | 4.8 |
| Conversion (mole %) | 5.2 | 99.8 | 99.3 | 99.6 |
| Selectivities | | | | |
| to carboxylic acid (mole %) | 81.2 | 95.9 | 93.5 | 96.1 |
| to aldehyde (mole %) | 8.6 | 0.48 | 1.1 | 0.53 |
| Yield of object carboxylic acid (mole %) | 4.2 | 95.7 | 92.8 | 95.7 |

CT=o-chlorotoluene
T=toluene
NT=m-nitrotoluene

The following examples 10 to 18 are not illustrative of the invention; they are intended to serve as reference or comparison examples.

### Example 10

In a 1.5 liter reactor made of titanium equipped with a reflux condenser, a stirrer, a heater, a gas inlet and a gas outlet, 400 g of acetic acid, 2.0 g of cobalt bromide (hexa-hydrate) (0.09 wt.% as Co and 0.24 wt.% as Br to acetic acid), 0.08 g of manganese acetate (tetra hydrate) (5.0 wt.% as Mn to cobalt component) and 267 g of o-chlorotoluene) were charged, and then nitrogen gas was fed from a gas inlet to give 10 Kg/cm$^2$ Gauge and the reactor was heated to 145°C by the heater. Then, air was fed from the gas inlet at an average flow rate of 74 N liter/hour under thorough stirring and the reaction was performed whilst maintaining the pressure to 10 Kg/cm$^2$ gauge and the temperature at 145°C and the concentration of oxygen in the discharged gas to 7 to 10% until the absorption of oxygen was substantially finished. The reaction time was 7.8 hours. After the reaction, the reaction mixture was cooled and the weight of the reaction mixture was 740 g.

The resulting reaction mixture was sampled and esterified and contents of the unreacted o-chlorotoluene, o-chlorobenzoic acid, o-chlorobenzaldehyde and benzoic acid were measured. As the results, conversion of o-chlorotoluene was 98.2 mole % and yield of o-chlorobenzoic acid was 95.4 mole %.

In the reaction mixture, contents of o-chlorobenzaldehyde and benzoic acid were respectively 0.21 mole % and 0.05 mole %.

### Example 11

In accordance with the process of Example 10 except using 200 g of o-chlorotoluene as the starting material, the reaction was performed. The results are shown in Table 3.

### Example 12

In accordance with the process of Example 10 except charging 1.4 g of cobalt bromide (hexahydrate) (0.063 wt.% as Co and 0.17 wt.% as Br to acetic acid), 0.56 g of manganese acetate (tetrahydrate) (50 wt.% as Mn to cobalt component), 0.6 g of hydrobromic acid (47 wt.% aq. sol.) as the catalyst, the reaction was performed. The results are shown in Table 3.

### Example 13

In accordance with the process of Example 10 except charging 1.0 g of cobalt bromide (hexahydrate) (0.045 wt.% as Co and 0.12 wt.% as Br to acetic acid), 0.80 g of manganese acetate (tetrahydrate) (100 wt.% as Mn to cobalt component) and 1.0 g of hydrobromic acid (47 wt.% aq. sol.) (0.12 wt.% as Br to acetic acid) as the catalyst, the reaction was performed. The results are shown in Table 3.

Example 14

In accordance with the process of Example 10 except charging 0.71 g of cobalt bromide (hexahydrate) (0.032 wt.% as Co and 0.087 wt.% as Br to acetic acid) 1.12 g of manganese acetate (196 wt.% as Mn to cobalt component) and 1.3 g of hydrobromic acid (47 wt.% aq. sol.) (15 wt.% as Br to acetic acid) as the catalyst, the reaction was performed. The results are shown in Table 3.

Example 15

In accordance with the process of Example 10 except charging 1.0 g of cobalt bromide (hexahydrate) (0.045 wt.% as Co and 0.12 wt.% as Br to acetic acid, 0.76 g of cobalt acetate (tetrahydrate) (0.045 wt.% as Co to acetic acid) and 0.08 g of manganese acetate (5.0 wt.% as Mn to cobalt component) as the catalyst, the reaction was performed. The results are shown in Table 4.

Example 16

In accordance with the process of Example 10, except adding 2.2 g of hydrobromic acid (0.47 wt.% aq. sol.) (0.26 wt.% as Br to acetic acid) to the catalyst of Example 14, the reaction was performed. The results are shown in Table 4.

Example 17

In accordance with the process of Example 10 except adding 1.5 g of cobalt acetate (tetrahydrate) (0.09 wt.% as Co to acetic acid) to the catalyst of Example 14, the reaction was performed. The results are shown in Table 4.

Example 18

In accordance with the process of Example 10 except reacting at 180°C under a pressure of 15 Kg/cm² gauge, the reaction was performed. The results are shown in Table 4.

TABLE 3

|  | Exp. 10 | Exp. 11 | Exp. 12 | Exp. 13 | Exp. 14 |
|---|---|---|---|---|---|
| Starting material | CT | CT | CT | CT | CT |
| Amount of o-CT (g) | 267 | 200 | 267 | 267 | 267 |
| Ratio of acetic acid/o-CT (by weight) | 1.5 | 2.0 | 1.5 | 1.5 | 1.5 |
| Catalyst |  |  |  |  |  |
| Co/acetic acid (wt.%) | 0.09 | 0.09 | 0.063 | 0.045 | 0.032 |
| Br/acetic acid (wt.%) | 0.24 | 0.24 | 0.24 | 0.24 | 0.24 |
| Mn/Co (Wt.%) | 5.0 | 5.0 | 50 | 100 | 196 |
| Reaction temp (°C) | 145 | 145 | 145 | 145 | 145 |
| Reaction pressure (Kg/cm²G) | 10 | 10 | 10 | 10 | 10 |
| Average air flow rate (N liter/hr.) | 74 | 103 | 68 | 65 | 61 |
| Reaction time (hrs) | 7.8 | 4.2 | 8.3 | 9.0 | 9.6 |
| Conversion of o-CT (mole %) | 98.2 | 99.5 | 99.0 | 98.6 | 97.3 |
| Yield of o-CBA (mole %) | 95.4 | 96.7 | 96.1 | 95.4 | 94.2 |
| o-chlorobenzaldehyde (mole %/o-CBA) | 0.21 | 0.20 | 0.22 | 0.25 | 0.42 |
| Benzoic acid (mole %/o-CBA) | 0.05 | 0.04 | 0.04 | 0.02 | 0.03 |

o-chlorotoluene=CT
o-chlorobenzoic acid=o-CBA

TABLE 4

| | Exp. 15 | Exp. 16 | Exp. 17 | Exp. 18 |
|---|---|---|---|---|
| Starting material | CT | CT | CT | CT |
| Amount of o-CT (g) | 267 | 267 | 267 | 267 |
| Ratio of acetic acid/o-CT (by weight) | 1.5 | 1.5 | 1.5 | 1.5 |
| Catalyst | | | | |
| Co/acetic acid (wt.%) | 0.09 | 0.09 | 0.18 | 0.09 |
| Br/acetic acid (wt.%) | 0.12 | 0.50 | 0.24 | 0.24 |
| Mn/Co (Wt.%) | 5 | 5 | 2.5 | 5 |
| Reaction temp (°C) | 145 | 145 | 145 | 180 |
| Reaction pressure (Kg/cm$^2$G) | 10 | 10 | 10 | 15 |
| Average air flow rate (N liter/hr.) | 61 | 88 | 102 | 191 |
| Reaction time (hrs) | 9.8 | 6.2 | 5.2 | 3.1 |
| Conversion of o-CT (mole %) | 98.4 | 98.9 | 99.3 | 98.1 |
| Yield of o-CBA (mole %) | 96.1 | 95.2 | 96.7 | 96.8 |
| o-chlorobenzaldehyde (mole %/o-CBA) | 0.44 | 0.20 | 0.17 | 0.20 |
| Benzoic acid (mole %/o-CBA) | 0.05 | 0.06 | 0.05 | 0.09 |

o-chlorotoluene=CT
o-chlorobenzoic acid=o-CBA

## Claims

1. A process for producing an aromatic monocarboxylic acid by oxidizing a benzene derivative having one alkyl group in the liquid phase with molecular oxygen or a molecular oxygen-containing gas in the presence of a catalyst and in the presence of a lower aliphatic monocarboxylic acid having 2 to 4 carbon atoms at an increased temperature, characterized in that said lower aliphatic monocarboxylic acid is used at a ratio of 0.15 to 0.5 times by weight of the amount of said benzene derivative present in the reaction system and the reaction is conducted at a temperature ranging from a temperature adequate to maintain the resulting aromatic monocarboxylic acid in liquid form to 180°C, said catalyst comprising a cobalt and manganese compound.

2. A process according to claim 1, characterized in that said lower aliphatic monocarboxylic acid is acetic acid, propionic acid or butyric acid.

3. A process according to one of claims 1 to 2, characterized in that said benzene derivative having one alkyl group is selected from alkylbenzenes, and substituted alkylbenzenes having at least one inert halogen, nitro, carboxymethyl or methoxy substituent on the benzene ring.

4. A process according to claim 3, characterized in that the benzene derivative having one alkyl group is chlorotoluene.

5. A process according to one of claims 1 to 4, characterized in that said catalyst is a compound of cobalt and manganese in combination with a reaction accelerator selected from halogens, halogen compounds, aldehydes and ketones.

6. A process according to claim 5, characterized in that said catalyst further comprises an additional nickel or cerium compound.

7. A process according to one of claims 5 or 6, characterized in that said reaction accelerator is a bromine compound.

8. A process according to one of claims 5 or 6, characterized in that the reaction accelerator is bromine.

9. A process according to claim 7, characterized in that the amount of the catalyst relative to the benzene derivative starting material ranges from 0.03 to 2 weight % as metal and that the manganese compound ranges from 0.5 to 500 weight % as manganese metal, based on said cobalt metal and that bromine or the bromine compound ranges from 0.05 to 5 weight % as bromine, based on said benzene derivative.

10. A process according to claim 7, characterized in that said benzene derivative is chlorotoluene and the amount of said catalyst comprising a cobalt compound and a manganese compound ranges from 0.05 to 0.5 wt.-% as metal based on said chlorotoluene, whereby the manganese compound ranges from 0.5 to 250 wt.% as manganese metal, based on said cobalt compound and the amount of bromine or the bromine compound ranges from 0.07 to 3 wt.-% as bromine, based on said chlorotoluene.

# 0 002 749

**Patentansprüche**

1. Verfahren zur Herstellung einer aromatischen Monocarbonsäure durch Oxidation eines Benzolderivatives mit einer Alkylgruppe in flüssiger Phase mit molekularem Sauerstoff oder einem molekularen Sauerstoff enthaltenden Gas in Gegenwart eines Katalysators und in Gegenwart einer niederaliphatischen Monocarbonsäure mit 2 bis 4 Kohlenstoffatomen bei erhöhter Temperatur, dadurch gekennzeichnet, daß die nieder-aliphatische Monocarbonsäure in einer Menge von dem 0,15- bis 0,5-fachen nach Gewicht der Menge des in dem Reaktionssystem vorliegenden Benzolderivatives verwendet wird und die Umsetzung bei einer Temperatur durchgeführt wird im Bereich von einer Temperatur, die ausreicht, die resultierende Monocarbonsäure in flüssiger Form zu halten, bis 180°C, wobei der genannte Katalysator eine Kobalt- und Manganverbindung umfaßt.

2. Verfahren nach Anspruch 1, dadurch gekennzeichnet, daß es sich bei der nieder-aliphatischen Monocarbonsäure um Essigsäure, Propionsäure oder Buttersäure handelt.

3. Verfahren nach irgendeinem der Ansprüche 1 oder 2, dadurch gekennzeichnet, daß das Benzolderivat mit einer Alkylgruppe ausgewählt ist unter Alkylbenzolen und substituierten Alkylbenzolen mit wenigstens einem inerten Halogen-, Nitro-, Carboxymethyl- oder Methoxysubstituenten am Benzolring.

4. Verfahren nach Anspruch 3, dadurch gekennzeichnet, daß es sich bei dem Benzolderivat mit einer Alkylgruppe um Chlortoluol handelt.

5. Verfahren nach irgendeinem der Ansprüche 1 bis 4, dadurch gekennzeichnet, daß der katalysator eine Verbindung des Kobalts und des Mangans in Kombination mit einem Reaktionsbeschleuniger, ausgewählt unter Halogen, Halogenverbindungen, Aldehyden und Ketonen, ist.

6. Verfahren nach Anspruch 5, dadurch gekennzeichnet, daß der Katalysator ferner eine zusätzliche Nickel- oder Cerverbindung umfaßt.

7. Verfahren nach irgendeinem der Ansprüche 5 oder 6, dadurch gekennzeichnet, daß der Reaktionsbeschleuniger eine Bromverbindung ist.

8. Verfahren nach irgendeinem der Ansprüche 5 oder 6, dadurch gekennzeichnet, daß der Reaktionsbeschleuniger Brom ist.

9. Verfahren nach Anspruch 7, dadurch gekennzeichnet, daß die Menge des Katalysators, bezogen auf das Benzolderivatausgangsmaterial im Bereich von 0,03 bis 2 Gew.-% als Metall beträgt und die der Manganverbindung im Bereich von 0,5 bis 500 Gew.-%, als Manganmetall, bezogen auf das Kobaltmetall, beträgt und die des Broms oder der Bromverbindung im Bereich von 0,05 bis 5 Gew.-%, als Brom, bezogen auf das Benzolderivat, beträgt.

10. Verfahren nach Anspruch 7, dadurch gekennzeichnet, daß das Benzolderivat Chlortoluol ist und die Menge des Katalysators, welcher eine Kobaltverbindung und eine Manganverbindung umfaßt, im Bereich von 0,05 bis 0,5 Gew.-%, als Metall, bezogen auf das Chlortoluol, beträgt, wobei die Manganverbindung im Bereich von 0,5 bis 250 Gew.-% als Manganmetall, bezogen auf die Kobaltverbindung, beträgt, und die Menge des Broms oder der Bromverbindung im Bereich von 0,07 bis 3 Gew.-% als Brom, bezogen auf das Chlortoluol, beträgt.

**Revendications**

1. Procédé de production d'un acide monocarboxylique aromatique par oxydation d'un dérivé du benzène qui contient un radical alkyle, en phase liquide avec de l'oxygène moléculaire ou un gaz contenant de l'oxygène moléculaire en présence d'un catalyseur et·en présence d'un acide monocarboxylique aliphatique inférieur comportant de deux à quatre atomes de carbone et à température élevée, caractérisé en ce que cet acide monocarboxylique aliphatique inférieur est utilisé en une quantité de 0,15 à 0,5 fois en poids la quantité dudit dérivé du benzène présent dans le système de réaction et en ce que la réaction est effectuée à une température allant d'une température adéquate pour maintenir l'acide monocarboxylique aromatique résultant à l'état liquide à 180°C, ledit catalyseur comprenant un composé de cobalt et de manganèse.

2. Procédé selon la revendication 1, caractérisé en ce que ledit acide monocarboxylique aliphatique inférieur est l'acide acétique, l'acide propionique ou l'acide butyrique.

3. Procédé selon l'une des revendications 1 ou 2, caractérisé en ce que ledit dérivé du benzène portant un radical alkyle est choisi parmi les alkylbenzènes et les alkylbenzènes substitués portant au moins un substituant inert halogène, nitro, carboxyméthyle ou méthoxy sur le noyau benzénique.

4. Procédé selon la revendication 3, caractérisé en ce que le dérivé du benzène portant un groupe alkyle est le chlorotoluène.

5. Procédé selon l'une des revendications 1 à 4, carctérisé en ce que ledit catalyseur est un composé de cobalt et de manganèse en combinaison avec un accélérateur de réaction choisi parmi les halogènes, les composés d'halogène, les aldéhydes et les cétones.

6. Procédé selon la revendication 5, caractérisé en ce que ledit catalyseur comprend en outre un composé supplémentaire de nickel ou de cérium.

7. Procédé selon l'une des revendications 5 ou 6, caractérisé en ce que ledit accélérateur de réaction est. un composé de brome.

11

**0 002 749**

8. Procédé selon l'une des revendications 5 ou 6, caractérisé en ce que ledit accélérateur de réaction est le brome.

9. Procédé selon la revendication 7, caractérisé en ce que la quantité du catalyseur par rapport au dérivé de benzène en qualité de matière première est de 0,03 à 2% en poids exprimés en métal, en ce que le composé de manganèse est utilisé à raison de 0,5 à 500% en poids exprimés en manganèse métallique, par rapport audit cobalt métallique et en ce que le brome ou le composé de brome est utilisé à raison de 0,05 à 5% en poids exprimés en brome par rapport audit dérivé de benzène.

10. Procédé selon la revendication 7, caractérisé en ce que ledit dérivé du benzène est le chlorotoluène et la quantité dudit catalyseur comprenant un composé de cobalt et un composé de manganèse est de 0,05 à 0,5% en poids exprimé en métal par rapport audit chlorotoluène, de sorte que le composé de manganèse est utilisé à raison de 0,5 à 250% en poids exprimés en manganèse métallique par rapport audit composé de cobalt, la quantité de brome ou du composé de brome étant de 0,07 à 3% en poids exprimés en brome, par rapport audit chlorotoluène.

12

# F I G. I

Selectivity to aromatic carboxylic acid (mole %)
Conversion of benzene derivative (mole %)

Selectivity to aldehyde (mole %)

Lower aliphatic monocarboxylic acid/benzene derivative
(ratio by weight)